(19) Europäisches Patentamt
European Patent Office
Office européen des brevets

(11) Veröffentlichungsnummer: **0 302 321 A2**

(12) # EUROPÄISCHE PATENTANMELDUNG

(21) Anmeldenummer: **88111818.6**

(22) Anmeldetag: **22.07.88**

(51) Int. Cl.[4]: **C07B 45/06 , C07C 149/34 , C07C 149/32 , C07C 149/28 , C07D 239/38**

(30) Priorität: **03.08.87 DE 3725640**

(43) Veröffentlichungstag der Anmeldung: **08.02.89 Patentblatt 89/06**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Hagemann, Hermann, Dr.**
**Kandinsky Strasse 52**
**D-5090 Leverkusen 1(DE)**
Erfinder: **Sasse, Klaus, Dr.**
**Pützweg 13**
**D-5060 Bergisch-Gladbach 2(DE)**
Erfinder: **Fischer, Reiner, Dr.**
**Rembrandtstrasse 15**
**D-4019 Monheim(DE)**

(54) **Verfahren zur Herstellung von Thiophenolen und neue Thiophenole.**

(57) Die Erfindung betrifft ein neues Verfahren zur Herstellung von neuen und bekannten Thiopenolen, indem man Halogenbenzole zunächst mit Natriumhydrogensulfid umsetzt und anschließend die Thiophenole durch Ansäuern freisetzt, sowie neue Thiophenole.

EP 0 302 321 A2

## Verfahren zur Herstellung von Thiophenolen und neue Thiophenole

Die Erfindung betrifft ein neues Verfahren zur Herstellung von neuen und bekannten Thiophenolen sowie neue Thiophenole.

Thiophenole können als Zwischenprodukte für die Synthese von herbiziden Wirkstoffen verwendet werden. Weiterhin lassen sich diese Verbindungen zu Herstellung von Farbstoffen, Pharmazeutika und Kautschukhilfsmitteln einsetzen. Die neuen und bekannten Thiophenole eignen sich besonders zur Herstellung von neuen Arylthioaminopyrimidinen, welche als Herbizide eingesetzt werden können.

Es ist bereits bekannt, daß man Thiophenole vom Typ der unten beschriebenen Verbindungender allgemeinen Formel (I) erhält, wenn man Halogenbenzole mit Alkalimetalldisulfiden im Verhältnis 1:1 bis 2:1, bevorzugt 2:1, umsetzt, anschließend ansäuert und den ausgefallenen Niederschlag mit Natriumsulfit-Lösung bei Temperaturen zwischen 90 und 100 °C digeriert. Nach Abtrennung des unlöslichen Bestandteils durch Filtration wird das Filtrat angesäuert, und man erhält das gewünschte Thiopenol in einer Ausbeute kleiner als 30%. Der mit Natriumsulfit-Lösung nicht extrahierbare Anteil der Reaktionsmischung wird dann mit wäßriger Natriumsulfid-Lösung gelöst, mit Säure zum Teil wieder ausgefällt und erneut mit Natriumsulfit-Lösung digeriert (vgl. DE-OS 2 156 345).

Der Nachteil dieses Verfahrens besteht darin, daß die Reaktionsschritte, also Zugabe von Natriumsulfit-Lösung usw., mehrmals wiederholt werden müssen, um bessere Ausbeuten zu erzielen. Eine derartige zeit- und materialaufwendige Reaktionsführung ist für eine technische Nutzung äußerst ungeeignet.

Weiterhin ist bekannt, daß man Thiophenole vom obigen Typ erhält, wenn man Thiophenole zunächst in die Thioessigsäurederivate überführt, in üblicher Weise nach Friedel-Crafts z.B. acetyliert oder benzoyliert und durch oxidative Spaltung die acylierten Thiophenole in Freiheit setzt. Um reine Produkte zu erhalten, müssen die dabei mitanfallenden Disulfane reduziert werden (J. Org. Chem 28, 3077-3082 (1963)).

Es sind ferner auch einige weitere Methoden zur Herstellung von Thiophenolen des obigen Typs geeignet, wie sie im Handbuch Houben-Weyl "Methoden der Organischen Chemie" Bd. E11 Teil 1, S. 32-62 (1985) für Thiophenole beschrieben sind, so z.B. solche Methoden, die über eine Diazotierung der entsprechenden Aminoverbindung verlaufen. Der Nachteil aller dieser Verfahren besteht darin, daß wie oben schon ausgeführt die Verfahrensweise mehrstufig und wesentlich aufwendiger ist und/oder daß vor allem nicht von den leicht zugänglichen Halogenbenzolen der Formel (II) ausgegangen werden kann.

Es wurde gefunden, daß man Thiophenole der Formel (I)

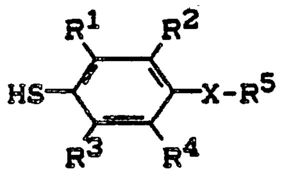

(I)

in welcher

$R^1$, $R^2$, $R^3$ und $R^4$ unabhängig voneinander jeweils für Wasserstoff, Halogen, Nitro, Alkyl, Alkoxy, Alkylthio, Alkoxyalkyl, Alkylthioalkyl, Halogenalkyl, Halogenalkoxy oder Alkylcarboxyl, jeweils gegebenenfalls substituiertes Aryl oder Hetaryl stehen,

$R^5$ für Alkyl, Alkoxyalkyl, Alkylthioalkyl, Halogenalkyl, Alkenyl, Halogenalkenyl, jeweils gegebenenfalls substituiertes Aryl oder Hetaryl steht und

X für Carbonyl, Sulfinyl oder Sulfonyl steht,

erhält, wenn man Halogenbenzole der Formel (II)

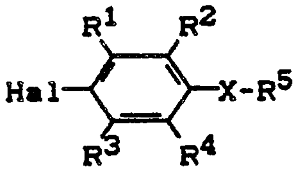

(II)

in welcher

$R^1$, $R^2$, $R^3$, $R^4$, $R^5$ und X die oben angegebene Bedeutung haben und

Hal für Halcgen steht,

zunächst mit Natriumhydrogensulfid oder Natriumsulfid in Gegenwart eines Verdünnungsmittels umsetzt zu den Verbindungen der Formel (III)

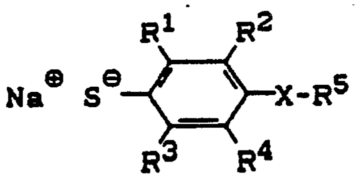

(III)

in welcher
$R^1$, $R^2$, $R^3$, $R^4$, $R^5$ und X die oben angegebene Bedeutung haben,
und anschließend Säure zusetzt.

Es ist als ausgesprochen überraschend anzusehen, daß man mit Hilfe des erfindungsgemäßen Verfahrens die gewünschten Thiophenole in hohen Ausbeuten und guter Reinheit erhält, da nach dem Stand der Technik zu erwarten war, daß in der alkalischen Reaktionsmischung, die stark nukleophilen Thiophenolatanionen mit dem noch nicht umgesetzten Halogenbenzol der Formel (II) unter Sulfanbildung reagieren (vgl. "Methoden der organischen Chemie" Houben-Weyl, Bd. E11, Teil 1, S. 158 ff).

Das erfindungsgemäße Verfahren weist eine Reihe von Vorteilen auf. Dazu gehört beispielsweise die einstufige Reaktionsführung und die einfache Aufarbeitung. Ein weiterer Vorteil des erfindungsgemäßen Verfahrens ist die leichte Zugänglichkeit der als Ausgangsverbindungen benötigten Halogenbenzole der Formel (II).

Die Kohlenstoffketten in den einzelnen Resten sind jeweils geradkettig oder verzweigt. Die Substitution der Reste kann jeweils einfach oder mehrfach, gleich oder verschieden erfolgen.

Mit Hilfe des erfindungsgemäßen Verfahrens erhält man vorzugsweise solche Thiophenole der Formel (I), in welcher
$R^1$, $R^2$, $R^3$ und $R^4$ unabhängig voneinander jeweils für Wasserstoff, Fluor, Chlor, Brom, Iod, Nitro, $C_1$-$C_6$-Alkyl, $C_1$-$C_6$-Alkoxy, $C_1$-$C_6$-Alkylthio, $C_1$-$C_4$-alkoxy-$C_1$-$C_6$-alkyl, $C_1$-$C_4$-Alkylthio-$C_1$-$C_6$-alkyl, Halogen-$C_1$-$C_6$-alkyl, Halogen-$C_1$-$C_6$-alkoxy oder $C_1$-$C_6$-Alkylcarboxyl, jeweils gegebenenfalls substituiertes Aryl mit 6 bis 10 Kohlenstoffatomen oder Hetaryl mit 5 bis 7 Ringatomen stehen, wobei als Aryl-und Hetaryl-Substituenten Halogen, Nitro, $C_1$-$C_4$-Alkyl, $C_1$-$C_4$-Alkoxy, $C_1$-$C_4$-Alkylthio und Halogen $C_1$-$C_4$-alkyl genannt seien und wobei das Hetaryl 1 oder 2 gleiche oder verschiedene Heteroatome wie Stickstoff, Schwefel oder Sauerstoff aufweist und insbesondere für durch oben genannte Substituenten substituiertes Pyridyl oder Pyrimidyl steht, und wobei Aryl insbesondere für durch oben genannte Substituenten substituiertes Phenyl oder Naphthyl steht,
$R^5$ für $C_1$-$C_{12}$-Alkyl, $C_1$-$C_6$-Alkoxy-$C_1$-$C_{12}$-alkyl, $C_1$-$C_6$-Alkylthio-$C_1$-$C_{12}$-alkyl, Halogen-$C_1$-$C_{12}$-alkyl, $C_2$-$C_{10}$-Alkenyl, Halogen-$C_2$-$C_{10}$-alkenyl, jeweils gegebenenfalls substituiertes Aryl mit 6 bis 10 Kohlenstoffatomen oder Hetaryl mit 5 oder 6 Ringatomen steht, wobei als Aryl- und Hetaryl-Substituenten Halogen, Nitro, $C_1$-$C_4$-Alkyl, $C_1$-$C_4$-Alkoxy, $C_1$-$C_4$-Alkylthio und Halogen-$C_1$-$C_4$-alkyl genannt seien und wobei das Hetaryl 1 oder 2 gleiche oder verschiedene Heteroatome wie Stickstoff, Schwefel oder Sauerstoff aufweist und insbesondere durch o.g. Substituenten substituiertes Pyridyl oder Pyrimidyl steht, wobei Aryl insbesondere für durch oben genannte Substituenten substituiertes Phenyl oder Naphthyl steht, und
X für Carbonyl, Sulfinyl oder Sulfonyl steht.

Besonders bevorzugt erhält man diejenigen Verbindungen der Formel (I), bei welchen
$R^1$, $R^2$, $R^3$ und $R^4$ unabhängig voneinander jeweils für Wasserstoff, Fluor, Chlor, Brom, Nitro, $C_1$-$C_4$-Alkyl, $C_1$-$C_4$-Alkoxy, $C_1$-$C_4$-Alkylthio, $C_1$-$C_2$-Alkoxy-$C_1$-$C_2$-alkyl, $C_1$-$C_2$-Alkylthio-$C_1$-$C_4$-alkyl, Halogen-$C_1$-$C_4$-alkyl, Halogen-$C_1$-$C_4$-alkoxy oder $C_1$-$C_4$-Alkylcarboxyl, jeweils gegebenenfalls substituiertes Phenyl, Naphthyl, Pyridyl oder Pyrimidyl steht, wobei als Substituenten Fluor, Chlor, Brom, Nitro, Methyl, Ethyl, Methoxy, Ethoxy, Methylthio, Ethylthio und Halogen-$C_1$-$C_2$-alkyl genannt seien,
$R^5$ für $C_1$-$C_{10}$-Alkyl, $C_1$-$C_4$-Alkoxy-$C_1$-$C_{10}$-alkyl, $C_1$-$C_4$-Alkylthio-$C_1$-$C_{10}$-alkyl, Halogen-$C_1$-$C_{10}$-alkyl, $C_2$-$C_5$-Alkenyl, Halogen-$C_2$-$C_5$-alkenyl, jeweils gegebenenfalls substiutiertes Phenyl, Pyridyl oder Pyrimidyl steht, wobei als Substituenten Fluor, Chlor, Brom, Nitro, Methyl, Ethyl, Methoxy, Ethoxy, Methylthio, Ethylthio und Halogen-$C_1$-$C_2$-alkyl (insbesondere Trifluormethyl) genannt seien, und
X für Carbonyl, Sulfinyl oder Sulfonyl steht.

Insbesondere erhält man die Verbindungen der Formel (I), in welcher
$R^1$ und $R^4$ unabhängig voneinander jeweils für Wasserstoff, Fluor, Chlor oder Methyl stehen,
$R^2$ und $R^3$ für Wasserstoff stehen,
$R^5$ für $C_1$-$C_9$-Alkyl, $C_1$-$C_2$-Alkoxy-$C_1$-$C_9$-alkyl, $C_1$-$C_2$-Alkylthio-$C_1$-$C_9$-alkyl, Halogen-$C_1$-$C_9$-alkyl, wobei Halo-

gen für Fluor und/oder Chlor steht, $C_2$-$C_4$-Alkenyl, Halogen-$C_2$-$C_4$-alkenyl, wobei Halogen für Fluor und/oder Chlor steht, gegebenenfalls substituiertes Phenyl, wobei als Substituenten Fluor, Chlor, Nitro, Methyl, Ethyl, Methoxy, Ethoxy und Trifluormethyl genannt seien,
und
X für Carbonyl, Sulfinyl oder Sulfonyl steht.

Verwendet man beispielsweise 4-(3,3-Dimethylbutyro)-Chlorbenzol und Natriumhydrogensulfid als Ausgangsstoffe, so läßt sich der Reaktionsablauf des erfindungsgemäßen Verfahrens durch das folgende Formelschema darstellen:

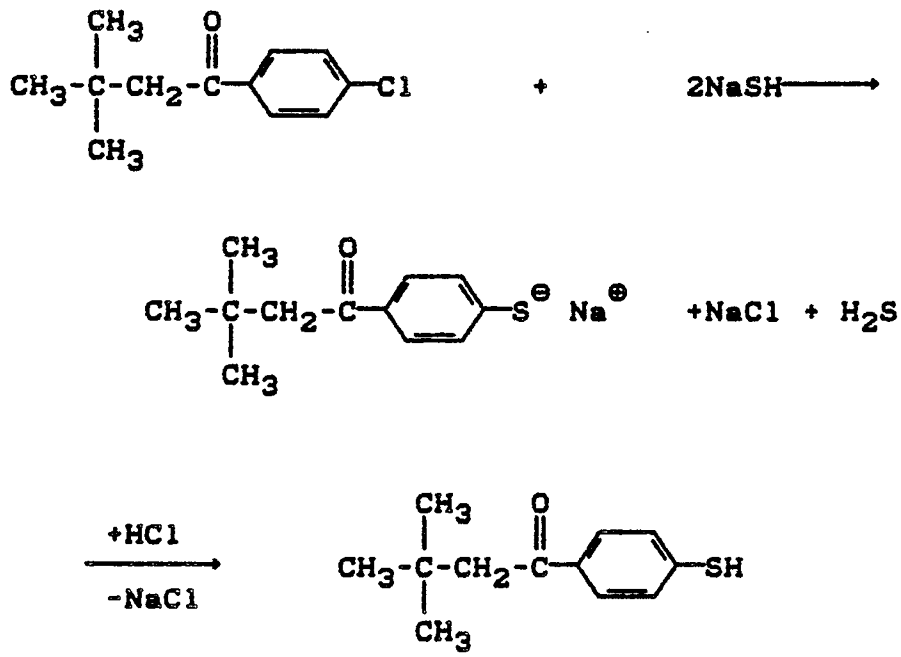


Die zur Durchführung des erfindungsgemäßen Verfahrens als Ausgangsstoffe benötigten Halogenbenzole sind durch die Formel (II) allgemein definiert. Bevorzugt sind Verbindungen der Formel (II), bei welchen $R^1$, $R^2$, $R^3$, $R^4$ $R^5$ und X für diejenigen Reste stehen, die bereits im Zusammenhang mit der Beschreibung der Stoffe der Formel (I) als bevorzugt bzw. besonders bevorzugt für diese Substituenten genannt wurden. Hal steht vorzugsweise für Fluor oder Chlor.

Die Halogenbenzole der Formel (II) sind bekannt oder lassen sich nach bekannten Verfahren, beispielsweise durch Friedel-Crafts-Acylierung, herstellen.

Das erfindungsgemäße Verfahren wird in Gegenwart eines geeigneten Verdünnungsmittels durchgeführt.

Als Verdünnungsmittel kommen praktisch alle inerten organischen Lösungsmittel in Frage. Hierzu gehören insbesondere N-Methylpyrrolidon, Dimethylformamid, Hexamethylphosphorsäuretriamid, Dimethylacetamid und N-Methylcaprolactam oder andere N,N-disubstituierte offenkettige oder cyclische Carbonsäureamide. Es können auch mehrere Lösungsmittel eingesetzt werden. Bevorzugt ist N-Methylpyrrolidon. Das oder die Lösungsmittel werden in einer solchen Menge eingesetzt, daß das Reaktionsgemisch gut rührbar ist.

Als Säuren kommen praktisch alle inerten anorganischen Säuren infrage, Vorzugsweise verwendet man Salzsäure oder verdünnte Schwefelsäure. Insbesondere verwendet man Salzsäure bei der Durchführung des erfindungsgemäßen Verfahrens.

Das erfindungsgemäße Verfahren wird im allgemeinen bei Tempreaturen zwischen 80 und 160 °C durchgeführt. Bevorzugt ist der Temperaturbereich zwischen 90 und 140 °C, insbesondere zwischen 100 und 125 °C. Die Umsetzungen werden im allgemeinen bei Normaldruck durchgeführt.

Zur Durchführung des erfindungsgemäßen Verfahrens setzt man im allgemeinen pro Mol an Halogenbenzol der Formel (II) 1 Mol bis 4 Mol, vorzugsweise 2 bis 3 Mol, insbesondere 2 bis 2,5 Mol an Natriumhydrogensulfid bzw. 1 Mol bis 4 Mol, vorzugsweise 1 bis 2 Mol, insbesondere 1 bis 1,5 Mol an Natriumsulfid ein.

Das erfindungsgemäße Verfahren wird vorzugsweise mit Natriumhydrogensulfid durchgeführt.

Die Reaktion wird so durchgeführt, daß man das Natriumhydrogensulfid bzw. Natriumsulfid in dem

entsprechenden Verdünnungsmittel vorlegt und beispielsweise durch Andestillieren mit Xylol oder Stickstoff bei Temeraturen von ca. 160 °C entwässert. Nach dem Abkühlen auf die Reaktionstemperatur wird das Halogenbenzol der Formel (II) zugegeben. Man läßt für einige Stunden (ca. 2 bis 6 Stunden) nachreagieren und destilliert anschließend das Lösungsmittel ab. Danach wird der Rückstand mit Wasser aufgenommen und das Filtrat mit Säure angesäuert und in üblicher Art und Weise aufgearbeitet (vgl. Herstellungsbeispiele).

Neu sind die Thiophenole der Formel (Ia),

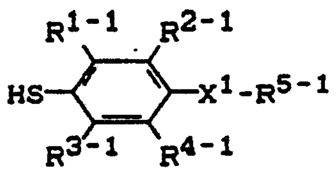

(Ia)

in welcher
$R^{1-1}$, $R^{2-1}$, $R^{3-1}$ und $R^{4-1}$ unabhängig voneinander jeweils für Wasserstoff, Halogen, Nitro, Alkyl, Alkoxy, Alkylthio, Alkoxyalkyl, Alkylthioalkyl, Halogenalkyl, Halogenalkoxy oder Alkylcarboxyl, jeweils gegebenenfalls substituiertes Aryl oder Hetaryl stehen,
$R^{5-1}$ für Alkyl, Alkoxyalkyl, Alkylthioalkyl, Halogenalkyl, Alkenyl, Halogenalkenyl, gegebenenfalls substituiertes Aryl oder gegebenenfalls substituiertes Hetaryl steht und
$X^1$ für Carbonyl, Sulfinyl oder Sulfonyl steht,
mit der Maßgabe, daß die Verbindungen 4-Acetylthiophenol, Bis-(4-thiophenyl)sulfon, 4-Chlor-4′-thio-benzophenon und 4-Chlor-4′-thiophenyl-phenylsulfon ausgenommen sind.

Die in der Formel (Ia) ausgenommenen Verbindungen sind aus DE-OS 2 156 345 und J.Org.Chem. 28, 3077-3082 (1963) bekannt.

Bevorzugt sind die Thiophenole der Formel (Ia),
in welcher
$R^{1-1}$, $R^{2-1}$, $R^{3-1}$ und $R^{4-1}$ unabhängig voneinander jeweils für Wasserstoff, Fluor, Chlor, Brom, Iod, Nitro, $C_1$-$C_6$-Alkyl, $C_1$-$C_6$-Alkoxy, $C_1$-$C_6$-Alkylthio, $C_1$-$C_4$-alkoxy-$C_1$-$C_6$-alkyl, $C_1$-$C_4$-Alkylthio-$C_1$-$C_6$-alkyl, Halogen-$C_1$-$C_6$-alkyl, Halogen-$C_1$-$C_6$-alkoxy oder $C_1$-$C_6$-Alkylcarboxyl, jeweils gegebenenfalls substituiertes Aryl mit jeweils 6 bis 10 Kohlenstoffatomen oder Hetaryl mit 5 bis 8 Ringatomen stehen, wobei als Aryl- und Hetaryl- Substituenten Halogen, Nitro, $C_1$-$C_4$-Alkyl, $C_1$-$C_4$-Alkoxy, $C_1$-$C_4$-Alkylthio und Halogen $C_1$-$C_4$-alkyl genannt seien und wobei das Hetaryl 1 oder 2 gleiche oder verschiedene Heteroatome wie Stickstoff, Schwefel oder Sauerstoff aufweist und insbesondere für durchoben genannte Substituenten substituiertes Pyridyl oder Pyrimidyl steht, und wobei Aryl insbesondere für durch oben genannte Substituenten substituiertes Phenyl oder Naphthyl steht,
$R^{5-1}$ für $C_1$-$C_{12}$-Alkyl, $C_1$-$C_6$-Alkoxy-$C_1$-$C_{12}$-alkyl, $C_1$-$C_6$-Alkylthio-$C_1$-$C_{12}$-alkyl, Halogen-$C_1$-$C_{12}$-alkyl, $C_2$-$C_{10}$-Alkenyl, Halogen-$C_2$-$C_{10}$-alkenyl, gegebenenfalls substituiertes Phenyl oder Naphthyl oder gegebenenfalls substituiertes Hetaryl mit 5 bis 7 Ringatomen, wobei als Phenyl-, Naphthyl- und Hetaryl-Substituenten $C_1$-$C_4$-Alkyl, $C_1$-$C_4$-Alkoxy, $C_1$-$C_4$-Alkylthio und Halogen-$C_1$-$C_4$-alkyl genannt seien und wobei des Hetaryl 1 oder 2 gleiche oder verschiedene Heteroatome wie Stickstoff, Schwefel oder Sauerstoff aufweist, insbesondere Hetaryl für durch oben genannte Substituenten substituiertes Pyridyl oder Pyrimidyl steht, und
X für Carbonyl, Sulfinyl oder Sulfonyl steht,
mit der Maßgabe, daß die Verbindungen 4-Acetylthiophenol, Bis-(4-thiophenyl)sulfon, 4-Chlor-4′-thiobenzophenon und 4-Chlor-4′-thiophenyl-phenylsulfon ausgenommen sind.

Besonders bevorzugt sind diejenigen Verbindungen der Formel (Ia),
in welcher
$R^{1-1}$, $R^{2-1}$, $R^{3-1}$ und $R^{4-1}$ unabhängig voneinander jeweils für Wasserstoff, Fluor, Chlor, Nitro, Methyl, Ethyl, n- oder iso-Propyl, Methoxy, Ethoxy, Methylthio, Ethylthio, Methoxymethyl, Methoxyethyl, Ethoxymethyl, Ethoxyethyl, Halogen-$C_1$-$C_2$-alkyl, Halogen-$C_1$-$C_2$-alkoxy, Methylcarboxyl, Ethylcarboxyl, n-Propylcarboxyl, iso-Propylcarboxyl, jeweils gegebenenfalls substituiertes Phenyl, Pyridyl oder Pyrimidyl steht, wobei als Substituenten jeweils Fluor, Chlor, Nitro, Methyl, Ethyl, Methoxy, Methylthio und/oder Trifluormethyl genannt seien,
$R^{5-1}$ für $C_1$-$C_{10}$-Alkyl, $C_1$-$C_4$-Alkoxy-$C_1$-$C_{10}$-alkyl, $C_1$-$C_4$-Alkylthio-$C_1$-$C_{10}$-alkyl, Halogen-$C_1$-$C_{10}$-alkyl, $C_2$-$C_5$-Alkenyl, Halogen-$C_2$-$C_5$-alkenyl, jeweils gegebenenfalls substituiertes Phenyl, Pyridyl oder Pyrimidyl steht, wobei als Substituenten Fluor, Chlor, Nitro, Methyl, Ethyl, Methoxy, Methylthio und/oder Trifluormethyl genannt seien und

X für Carbonyl, Sulfinyl oder Sulfonyl steht,
mit der Maßgabe, daß die Verbindungen 4-Acetylthiophenol, Bis-(4-thiophenyl)sulfon, 4-Chlor-4′-thiobenzo phenon und 4-Chlor-4′-thiophenyl-phenylsulfon ausgenommen.

Ganz besonders bevorzugt sind die Verbindungen der Formel (Ia),
in welcher
$R^{1-1}$, $R^{2-1}$, $R^{3-1}$ und $R^{4-1}$ unabhängig voneinander jeweils für Wasserstoff, Fluor, Chlor, Methyl, Methoxy, Methylthio oder Trifluormethyl steht,
$R^{5-1}$ für Methyl, Ethyl, n- oder iso-Propyl, n-, iso-, sec.- oder tert.-Butyl, n-Pentyl, iso-Pentyl, neo-Pentyl, n-Hexyl, iso-Hexyl, neo-Hexyl, n-Heptyl, iso-Heptyl, neo-Heptyl, Methylcarboxyl, Ethylcarboxyl, Allyl, Methylvinyl, gegebenenfalls einfach bis dreifach gleich oder verschieden durch Fluor, Chlor, Methyl, Methoxy oder Trifluormethyl substituiertes Phenyl,
$X^1$ für Carbonyl, Sulfinyl oder Sulfonyl steht,
mit der Maßgabe, daß die Verbindung 4-Acetyl-thiophenol ausgenommen ist und daß $R^{5-1}$ nicht für 4-Chlorphenyl steht.

Sowohl die neuen als auch die bekannten Thiophenole der Formel (I) sind wertvolle Zwischenprodukte und eignen sich beispielseweise zur Synthese von herbizid wirksamen Aryloxy (bzw. thio)aminopyrimidinen, welche in parallel eingereichten Patentanmeldungen beschrieben sind.

So erhält man neue Aryloxy (bzw. thio)aminopyrimidine der Formel (IV)

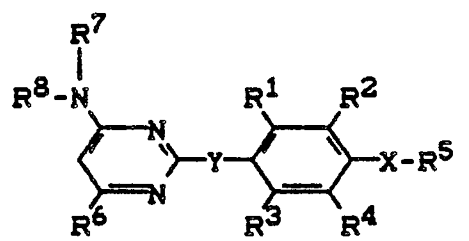

(IV)

in welcher
$R^1$, $R^2$, $R^3$, $R^4$, $R^5$ und X die oben angegebene Bedeutung haben,
$R^6$ für Wasserstoff oder gegebenenfalls durch Halogen, Alkoxy und/oder Alkylthio substituiertes Alkyl steht,
$R^7$ für Wasserstoff, Alkyl, Alkoxyalkyl, Cycloalkyl oder Alkenyl steht,
$R^8$ für Wasserstoff oder Alkyl steht oder
$R^7$ und $R^8$ gemeinsam mit dem Stickstoffatom, an das sie gebunden sind, einen drei- bis sechsgliedrigen Ring mit 1 oder 2 weiteren gleichen oder verschiedenen Heteroatomen wie Stickstoff, Sauerstoff oder Schwefel bilden können,
und
Y für Sauerstoff oder Schwefel steht,
wenn man Pyrimidinderivate der Formel (V)

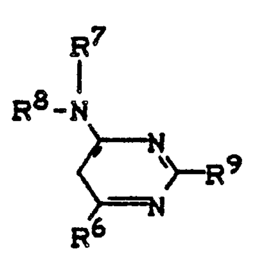

(V)

in welcher
$R^6$, $R^7$ und $R^8$ die oben angegebene Bedeutung haben und
$R^9$ für Halogen oder Alkylsulfonyl, insbesondere Chlor, Methylsulfonyl oder Ethylsulfonyl, steht,
mit Thiophenolen der Formel (I)

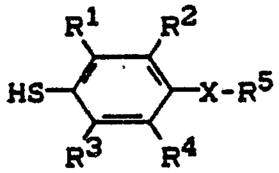

(I)

in welcher
$R^1$, $R^2$, $R^3$, $R^4$, $R^5$ und X die oben angegebene Bedeutung haben,
oder mit Thiophenolen der Formel (VI)

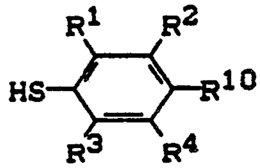

(VI)

in welcher
$R^1$, $R^2$, $R^3$, und $R^4$ die oben angegebene Bedeutung haben
und
$R^{10}$ für Alkyl, Alkoxyalkyl, Alkylthioalkyl, Halogenalkyl, Alkenyl oder Halogenalkenyl steht,
gegebenenfalls in Gegenwart eines Verdünnungsmittels wie beispielsweise N-Methylpyrrolidon sowie gegebenenfalls in Gegenwart eines Säurebindemittels wie beispielsweise Natrium- oder Kaliumhydroxid bei Temperaturen zwischen 50 °C und 150 °C umsetzt.

Bei der Durchführung dieses Verfahrens setzt man die die Reaktanten der Formeln (V) bzw. (VI) und (I) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden (vgl. Herstellungsbeispiele).

Die Pyrimidinderivate der Formel (V) sind bekannt oder können nach bekannten Verfahren der organischen Chemie erhalten werden (vgl. A. Weisberger: The Chemistry of Heterocyclic Compounds; The Pyrimidines, 1962 Interscience, New York).

Die Thiophenole der Formel (VI) erhält man, wenn man erfindungsgemäße Thiophenole der Formel (Ib)

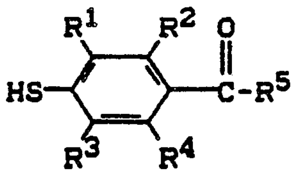

(Ib)

in welcher
$R^1$, $R^2$, $R^3$, $R^4$ und $R^5$ die oben angegebene Bedeutung haben,
mit Reduktionsmitteln wie beispielsweise Natriumborhydrid in Gegenwart eines Verdünnungsmittels wie beispielsweise Diethylenglykoldimethylether bei Temperaturen zwischen 20 °C und 140 °C umsetzt (vgl. Herstellungsbeispiele).

Die erfindungsgemäßen Thiophenole der Formel (Ib) fallen unter die Formel (I) (X = Carbonyl) und sind dort beschrieben.

## Herstellungsbeispiele

### Beispiel I-1

2-Chlor-4-acetyl-thiophenol

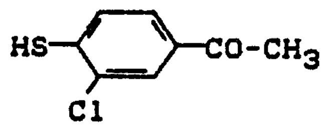

Es werden 110 g (1,48 Mol) Natriumhydrogensulfid ($NaSH \cdot H_2O$) zusammen mit 1 l N-Methylpyrrolidon vorgelegt und mit Stickstoff bei 160 °C entwässert. Nach Abkühlung auf 140 °C gibt man 112 g (0,59 Mol) 3,4-Dichloracetophenon hinzu, läßt 3 Stunden bei 160 °C nachreagieren und destilliert das N-Methylpyrrolidon ab. Der Rückstand wird in 1 l Wasser aufgenommen, filtriert und das Filtrat bei 0-10 °C mit halbkonzentrierter Salzsäure angesäuert. Der Niederschlag wird abgesaugt, neutral gewaschen und getrocknet. Destillation unter vermindertem Druck ergibt 99,8 g (90,7 % der Theorie) 2-Chlor-4-acetyl-thiopenol vom Siedepunkt: 125-130 ° C/0,1 mbar.

Beispiel I-2

4-(neo-Pentylcarbonyl)-thiophenol

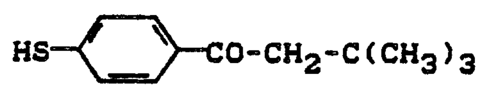

Es werden 18,5 g (0,2 Mol) Natriumhydrogensulfid ($NaSH \cdot H_2O$) in 100 ml N-Methylpyrrolidon vorgelegt und durch Andestillieren mit Xylol entwässert. Man läßt auf 140 °C abkühlen, gibt 21,05 g (0,1 Mol) 4-(neo-Pentylcarbonyl)-chlorbenzol hinzu, läßt 3 Stunden bei 160 °C nachreagieren und arbeitet, wie im Beispiel I-1 beschrieben, auf.
Man erhält 14 g (70 % der Theorie) des gewünschten Produkts vom Siedepunkt: 110-115 °C/ 0,1 mbar.

Beispiel I-3

4-(neo-Pentylsulfonyl)-thiophenol

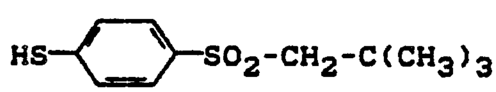

Es wurden 14,4 g (0,194 Mol) Natriumhydrogensulfit ($NaSH \cdot H_2O$) in 120 ml N-Methylpyrrolidon vorgelegt und mit Stickstoff bei 160 °C entwässert. Man läßt auf 100 °C abkühlen, gibt 20 g (0,081 Mol) 4-(neo-Pentylsulfonyl)-chlorbenzol hinzu, läßt 5 Stunden bei 125 °C nachreagieren und detilliert das Lösungsmittel unter vermindertem Druck ab. Der Rückstgand wird in 150 ml Wasser gelöst und bei 10-20 °C mit halbkonzentrierter Salzsäure sauer gestellt. Man extrahiert mit 100 ml Methylenchlorid, wäscht die organische Phase mit Wasser neutral, trocknet mit Magnesiumsulfat, engt ein, gibt 50 ml Toluol hinzu und destilliert bis zu einer Innentemperatur von 130 °C bei 0,1 mbar an.

Man erhält 19 g 4-(neo-Pentylsulfonyl)-thiophenol vom Siedepunkt: 130 °C/0,1 mbar; Reinheit (Gaschromatographie) : 98,6 % = 95 % der Theorie.

Analog den Beispielen I-1 bis I-3 und entsprechend den allgemeinen Angaben zur Herstellung erhält

man die folgenden Thiophenole der Formel (I):

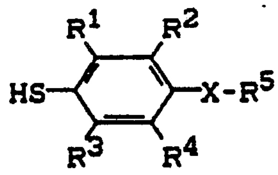

(I)

**Tabelle 1**

| Beispiel Nr. | $R^1$ | $R^2$ | $R^3$ | $R^4$ | $R^5$ | X | Siedepunkt (°C/mbar)/ Schmelzpunkt °C |
|---|---|---|---|---|---|---|---|
| 4 | H | H | H | H | $-CH_3$ | -CO- | 80- 82/0,1 |
| 5 | H | H | H | H | $-CH_2CH_2CH_3$ | -CO- | 104-106/0,2 |
| 6 | H | H | H | H | $-CH_2CH(CH_3)_2$ | -CO- | 93- 95/ 0,1 |
| 7 | H | H | H | H | $-CH(CH_3)_2$ | -CO- | 74 -75 |
| 8 | Cl | H | H | H | $-CH_2CH_2COOH$ | -CO- | 129-130 |
| 9 | Cl | H | H | $CH_3$ | $-CH_3$ | -CO- | 88- 90 |
| 10 | H | H | H | H | 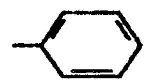 | -CO- | 70- 71 |
| 11 | Cl | H | H | H | 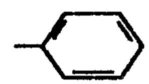 | -CO- | 71- 72 |
| 12 | H | H | H | H | 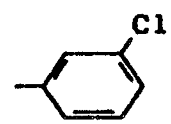 | -CO- | 107-108 |

**Tabelle 1 (Fortsetzung)**

| Beispiel Nr. | $R^1$ | $R^2$ | $R^3$ | $R^4$ | $R^5$ | X | Siedepunkt (°C/mbar)/ Schmelzpunkt °C |
|---|---|---|---|---|---|---|---|
| 13 | H | H | H | H | $-C(CH_3)=CH_2$ | -CO- | 85- 87/0,1 |
| 14 | H | H | H | H | $-CH_2C(CH_3)_3$ | -SO- | 112-116 |
| 15 | H | H | H | H | $-CH_2CH_2C(CH_3)_3$ | -CO- | |

Herstellungsbeispiel für herbizid wirksame Verbindungen:

Beispiel IV-1

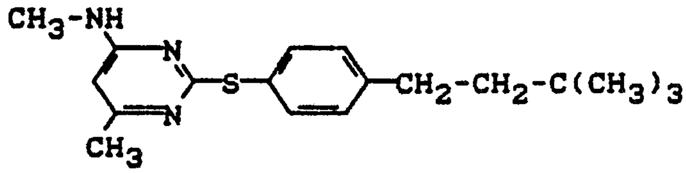

3,7 g (17 mmol) 4-Neohexyl-thiophenol werden in 20 ml N-Methylpyrrolidon unter Stickstoffatmosphäre mit 1,18 g (21 mmol) pulverisiertem Kaliumhydroxid versetzt und 15 Minuten verrührt. Nach Zugabe von 3,42 g (17 mmol) 4-Methylamino-6-methyl-2-methylsulfonyl-pyrimidin wird 90 Minuten auf 120 °C erwärmt. Die Reaktionsmischung wird in 150 ml 1N Natriumhydroxid-Lösung gegossen, mit Toluol extrahiert, getrocknet und im Vakuum eingedampft. Den Rückstand chromatographiert man mit Cyclohexan/Essigester 3:1 und erhält nach Kristallisation aus Ether/n-Hexan 3,23 g (60,3 % der Theorie) des gewünschten Produkts vom Schmelzpunkt 105 °C.

Beispiel IV-2

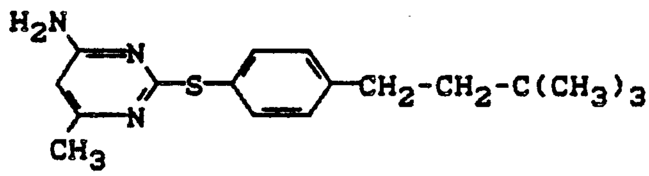

In analoger Weise wie unter Beispiel IV-1 beschrieben, erhält man das Produkt IV-2 in einer Ausbeute von 2,7 g (44,9% der Theorie) vom Schmelzpunkt 111 °C.

Beispiel VI-1

4-(neo-Hexyl)-thiophenol

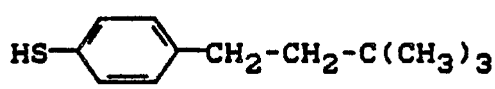

Es werden 7,5 g (0,2 Mol) Natriumborhydrid in 140 ml Diethylglykoldimethylether vorgelegt und bei 30 - 40 °C eine Lösung von 41,6 g (0,2 Mol) 4-(neo-Pentylcarbonyl)-thiophenol in 60 ml Diethylglykoldimethylether zugetropft. Man läßt 2 Stunden bei 120 °C nachreagieren, gibt unter Eiskühlung 100 ml Wasser zu, stellt mit 20 %iger Schwefelsäure sauer und gibt nochmal 200 ml Wasser zu. Die Reaktionsmischung wird nun mit 250 ml Methylenchlorid extrahiert, die organische Phase mit 250 ml Wasser ausgeschüttelt, über Magnesiumsulfat getrocknet und über eine Ringspaltkolonne destilliert. Man erhält 23 g (60 % d.Th.) 4-(neo-Hexyl)-thiophenol vom Siedepunkt 98 °C/0,4 mbar.

Anwendungsbeispiel

In dem nachfolgenden Anwendungsbeispiel wurde die nachstehend aufgeführte Verbindung als Vergleichssubstanz eingesetzt:

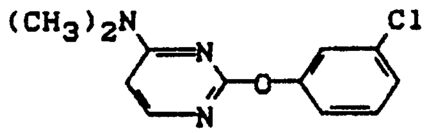

(A)

2-(3-Chlorphenoxy)-4-dimethylaminopyrimidin
(bekannt aus EP-A 1187/Beispiel 28)

Beispiel A

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen z.B. die Verbindungen gemäß den Herstellungsbeispielen IV-1 und IV-2 eine deutlich bessere herbizide Wirkung gegen Unkräuter als die Vergleichssubstanz (A).

**Tabelle A** — Post-emergence-Test / Gewächshaus

| Wirkstoff | Wirkstoffaufwand g/ha | Weizen | Amaranthus | Chenopodium | Galinsoga | Sinapis | Setaria |
|---|---|---|---|---|---|---|---|
| A (bekannt aus EP-A 1 187) | 250 | 0 | 0 | 0 | 0 | 0 | 0 |
| IV-1 | 125 | 10 | 100 | 90 | 100 | 100 | 90 |
| IV-2 | 125 | 10 | 100 | 100 | 100 | 100 | 100 |

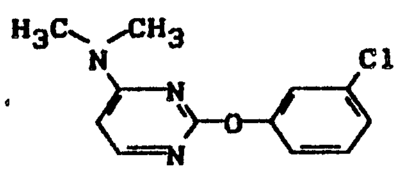

(A)

(bekannt aus EP-A-1 187, Bsp. 28)

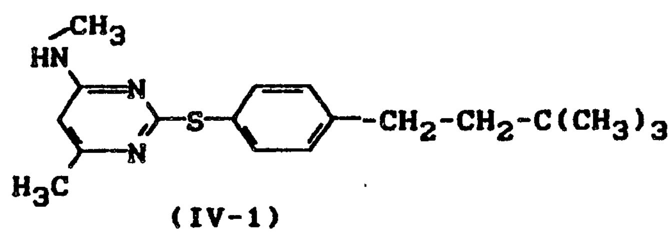

(IV-1)

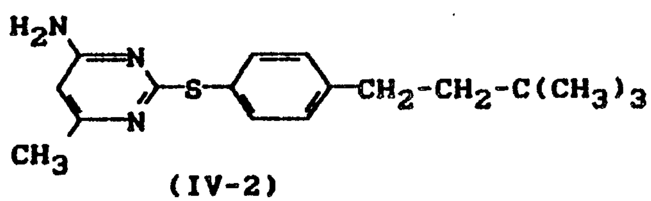

(IV-2)

## Ansprüche

1. Verfahren zur Herstellung von Thiophenolen der Formel (I)

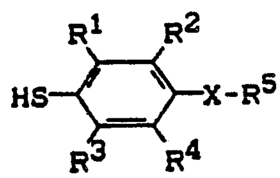

(I)

in welcher
$R^1$, $R^2$, $R^3$ und $R^4$ unabhängig voneinander jeweils für Wasserstoff, Halogen, Nitro, Alkyl, Alkoxy, Alkylthio, Alkoxyalkyl, Alkylthio-alkyl, Halogenalkyl, Halogenalkoxy oder Alkylcarboxyl, jeweils gegebenenfalls substituiertes Aryl oder Hetaryl stehen,
$R^5$ für Alkyl, Alkoxyalkyl, Alkylthioalkyl, Halogenalkyl, Alkenyl, Halogenalkenyl, jeweils gegebenenfalls substituiertes Aryl oder Hetaryl steht und
X für Carbonyl, Sulfinyl oder Sulfonyl steht,
dadurch gekennzeichnet, daß man Halogenbenzole der Formel (II)

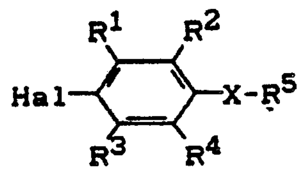

(II)

in welcher
$R^1$, $R^2$, $R^3$, $R^4$, $R^5$ und X die oben angegebene Bedeutung haben und
Hal für Halogen steht,
zunächst mit Natriumhydrogensulfid oder Natriumsulfid in Gegenwart eines Verdünnungsmittels umsetzt zu den Verbindungen der Formel (III)

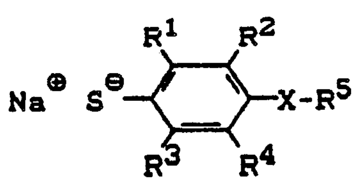

(III)

in welcher
$R^1$, $R^2$, $R^3$, $R^4$, $R^5$ und X die oben angegebene Bedeutung haben,
und anschließend Säure zusetzt.

2. Verfahren nach Anpruch 2, dadurch gekennzeichnet daß man die Umsetzung bei Temperaturen zwischen 80 und 160 °C durchführt.

3. Verfahren nach Anspruch 1, dadurch gekennzeichnet daß man Salzsäure oder verdünnte Schwefelsäure als Säure zusetzt.

4. Verfahren nach Anspruch 1, dadurch gekennzeichnet, daß man auf 1 Mol an Halogenbenzol der Formel (II) 1 bis 4 Mol Natriumhydrogensulfid einsetzt.

5. Verfahren nach Anspruch 1, dadurch gekennzeichnet, daß man auf 1 Mol an Halogenbenzol der Formel (II) 1 bis 4 Mol Natriumsulfid einsetzt.

6. Thiophenole der Formel (Ia),

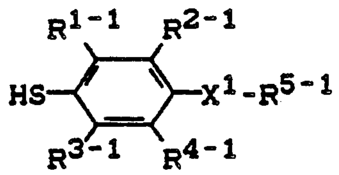

(Ia)

in welcher
$R^{1-1}$, $R^{2-1}$, $R^{3-1}$ und $R^{4-1}$ unabhängig voneinander jeweils für Wasserstoff, Halogen, Nitro, Alkyl, Alkoxy, Alkylthio, Alkoxyalkyl, Alkylthioalkyl, Halogenalkyl, Halogenalkoxy oder Alkylcarboxyl, jeweils gegebenenfalls substituiertes Aryl oder Hetaryl stehen,
$R^{5-1}$ für Alkyl, Alkoxyalkyl, Alkylthioalkyl, Halogenalkyl, Alkenyl, Halogenalkenyl, gegebenenfalls substituiertes Aryl oder gegebenenfalls substituiertes Hetaryl steht und
$X^1$ für Carbonyl, Sulfinyl oder Sulfonyl steht,
mit der Maßgabe, daß die Verbindungen 4-Acetyl-thiophenol, Bis-(4-thiophenyl)sulfon, 4-Chlor-4′-thiobenzophenon und 4-Chlor-4′-thiophenyl-phenylsulfon ausgenommen sind.

7. Thiophenole der Formel (Ia) gemäß Anspruch 6
in welcher
$R^{1-1}$, $R^{2-1}$, $R^{3-1}$ und $R^{4-1}$ unabhängig voneinander jeweils für Wasserstoff, Fluor, Chlor, Brom, Iod, Nitro, $C_1$-$C_6$-Alkyl, $C_1$-$C_6$-Alkoxy, $C_1$-$C_6$-Alkylthio, $C_1$-$C_4$-alkoxy-$C_1$-$C_6$-alkyl, $C_1$-$C_4$-Alkylthio-$C_1$-$C_6$-alkyl, Halogen-$C_1$-$C_6$-alkyl, Halogen-$C_1$-$C_6$-alkoxy oder $C_1$-$C_6$-Alkylcarboxyl, jeweils gegebenenfalls substituiertes Aryl mit 6 bis 10 Kohlenstoffatomen oder Hetaryl mit 5 bis 8 Ringatomen stehen, wobei als Aryl-und Hetaryl-Substituenten Halogen, Nitro, $C_1$-$C_4$-Alkyl, $C_1$-$C_4$-Alkoxy, $C_1$-$C_4$-Alkylthio und Halogen-$C_1$-$C_4$-alkyl genannt seien und wobei das Hetaryl 1 oder 2 gleiche oder verschiedene Heteroatome wie Stickstoff, Schwefel oder Sauerstoff aufweist,
$R^{5-1}$ für $C_1$-$C_{12}$-Alkyl, $C_1$-$C_6$-Alkoxy-$C_1$-$C_{12}$-alkyl, $C_1$-$C_6$-Alkylthio-$C_1$-$C_{12}$-alkyl, Halogen-$C_1$-$C_{12}$- alkyl, $C_2$-$C_{10}$-Alkenyl, Halogen-$C_2$-$C_{10}$-alkenyl, jeweils gegebenenfalls substituiertes Phenyl oder Naphthyl oder gegebenenfalls substituiertes Hetaryl mit 5 bis 7 Ringatomen steht, wobei als Phenyl-, Naphthyl- und Hetaryl-Substituenten $C_1$-$C_4$-Alkyl, $C_1$-$C_4$-Alkoxy, $C_1$-$C_4$-Alkylthio und Halogen-$C_1$-$C_4$-alkyl genannt seien und wobei das Hetaryl 1 oder 2 gleiche oder verschiedene Heteroatome wie Stickstoff, Schwefel oder Sauerstoff aufweist, und
X für Carbonyl, Sulfinyl oder Sulfonyl steht,
mit der Maßgabe, daß die Verbindungen 4-Acetylthiophenol, Bis-(4-thiophenyl)sulfon, 4-Chlor-4′-thiobenzophenon und 4-Chlor-4′-thiophenyl-phenyl-sulfon ausgenommen sind.